# EUROPÄISCHE PATENTANMELDUNG

(11) **EP 1 435 518 A2**
(43) Veröffentlichungstag der Anmeldung: **07.07.2004**
(21) Anmeldenummer: 03029104.1
(22) Anmeldetag: 17.12.2003
(51) Int. Cl.: G01N 33/36

(54) **Lebensdauerprüfung und entsprechende Vorrichtung für Bügeltextilien**

(30) Priorität: 30.12.2002 DE 10261354
(71) Anmelder: BSH Bosch und Siemens Hausgeräte GmbH, 81739 München (DE)
(72) Erfinder: Hafer, Christian, Dr., 85435 Erding (DE); Redlin, Kathrin, 14050 Berlin (DE); Srama, Uwe, 12209 Berlin (DE)

(57) **Zusammenfassung**

Die Lebensdauer von Blähsäcken für Bügelmaschinen beziehungsweise sogenannten Bügelpuppen soll abgeschätzt werden. Hierzu wird eine Vorrichtung vorgeschlagen, die einen befüllbaren Formkörper (1) und eine Befüllungseinrichtung, mit der der befüllbare Formkörper (1) mit einem Medium, insbesondere Luft, wiederholt befüllbar und leerbar ist, aufweist. Weiterhin kann ein Steuergerät vorgesehen sein, um eine Vielzahl von Bügelzyklen mit Befüllungs- und Entleerungsphasen des Formkörpers zu simulieren. Darüber hinaus können weitere Belastungen wie Waschen, Kälte/Wärme-Behandlung, Schadgasbelastung und dergleichen durchgeführt werden.

## Beschreibung

Die vorliegende Erfindung betrifft eine Vorrichtung zur Bestimmung der Lebensdauer eines befüllbaren, textilen Formkörpers für eine Bügelvorrichtung. Ferner betrifft die vorliegende Erfindung ein entsprechendes Verfahren zur Bestimmung der Lebensdauer eines befüllbaren, textilen Formkörpers.

Industrielle Bügelmaschinen, die beispielsweise zum Bügeln von Hemden und Blusen verwendet werden sollen, bestehen gegebenenfalls aus einer sogenannten "Bügelpuppe", die in etwa die Form des Bügelguts besitzt und aufblasbar ist. Das Bügelgut wird schleuderfeucht auf diese Bügelpuppe aufgebracht und diese wird anschließend mit erhitzter Luft aufgeblasen. Die erhitzte Luft durchdringt die aus Textilmaterial bestehende Bügelpuppe und das Bügelgut. Durch den Luftdruck in der Bügelpuppe und die aufgebrachte Wärme wird das Bügelgut getrocknet und geglättet.

Wird ein Textil für die Bügelpuppe oder einen Blähsack einer derartigen Bügelmaschine verwendet, wird es Belastungen ausgesetzt, die so für Bekleidungs- und Textilindustrie nicht bekannt sind. Auch ist es unüblich, Lebensdauern für Textilien anzugeben, da die Belastungen durch zum Beispiel Tragen von Bekleidung zu undefiniert ist, um eine solche Angabe seriös zu machen.

Soll die oben beschriebene Bügelmaschine mit Blähsack oder Bügelpuppe aus Textilmaterial als Haushaltsgerät eingesetzt werden, so ist eine Prüfung der Lebensdauer unerlässlich, da der Kunde für ein solches Gerät eine bestimmte Lebensdauer erwartet und gewisse Garantiezeiten gewährleistet werden müssen.

Für eine Lebensdauerbestimmung von Textilien liegen jedoch kaum Erfahrungswerte vor. Erschwert wird eine derartige Bestimmung, wenn ein Textil eingesetzt werden soll, das auch für den vorgesehenen Einsatzbereich noch unbekannt ist. Um für ein solches Textil eine qualifizierte Aussage zu treffen, wäre ein Durchlauf der Lebensdauer nötig. Bei Haushaltsgeräten setzt man jedoch Lebensdauern von 10 Jahren voraus, weshalb eine entsprechende Prüfung unter realen Bedingungen selbst bei Zeitraffung noch zu langwierig wäre.

Die Aufgabe der vorliegenden Erfindung besteht somit darin, eine Vorrichtung und ein Verfahren zur Lebensdauerprüfung von Textilien vorzuschlagen.

Erfindungsgemäß wird diese Aufgabe gelöst durch eine Vorrichtung zur Bestimmung der Lebensdauer eines befüllbaren, textilen Formkörpers für eine Bügelvorrichtung mit einer Befüllungseinrichtung, mit der der befüllbarene Formkörper mit einem Medium, insbesondere Luft, wiederholt befüllbar und leerbar ist.

Ferner ist erfindungsgemäß vorgesehen ein Verfahren zur Bestimmung der Lebensdauer eines befüllbaren, textilen Formkörpers für eine Bügelvorrichtung durch wiederholtes Befüllen und Entleeren des befüllbaren Formkörpers mit einem Medium, insbesondere Luft.

Damit ist es möglich, den wesentlichen Belastungsvorgang, der bei industriellen Bügelmaschinen auftritt, nämlich das Auffüllen eines textilen Formkörpers beziehungsweise einer Bügelpuppe zu simulieren. Dabei kann festgestellt werden, wie oft ein derartiger Befüllungsvorgang ohne Beschädigung des Formkörpers möglich ist.

Vorzugsweise ist eine Messvorrichtung vorgesehen, um mindestens eine Materialeigenschaft des Bügelguts nach einer gewissen Behandlung beziehungsweise Zyklenzahl zu messen. Derartige Messungen betreffen in erster Linie die Luftdurchlässigkeit, die Höchstzugkraft beziehungsweise die Höchstzugkraftdehnung des Textilmaterials des Formkörpers. Mit einer entsprechenden Auswerteeinrichtung kann aus der gemessenen, mindestens einen Materialeigenschaft die Lebensdauer des Formkörpers bestimmt werden. Weiterhin kann eine Messeinrichtung zum Messen von Prozessbedingungen, wie Temperatur, Druck oder Feuchtigkeit des Befüllungs - Mediums vorgesehen werden, um die Prozessbedingungen zu überwachen, insbesondere nachzusteuern und zu dokumentieren.

Vorzugsweise ist eine Befüllungseinrichtung vorgesehen, mit der die einzufüllende Luft klimatisiert werden kann. Weiterhin kann vorgesehen sein, dass die Befüllungseinrichtung die einzufüllende Luft mit Gasen und/oder Dämpfen versetzt. Damit können die klimatischen Bedingungen, die in der Realität beim Bügeln auftreten, hinsichtlich der verwendeten erhitzten Luft und der Umgebungsbedingungen sowie der verwendeten Zusatzmittel simuliert werden.

Auf dem Formkörper beziehungsweise der Bügelpuppe kann ein Bügelgut aufgebracht sein. Dies dient zur Simulation der mechanischen Reibung, die bei jedem Aufblasvorgang zwischen dem Bügelgut und dem sich aufblähenden Formkörper entsteht und diesen im Laufe der Zeit mechanisch beansprucht.

Vorteilhafterweise umfasst die erfindungsgemäße Vorrichtung zur Bestimmung der Lebensdauer eines textilen Formkörpers auch eine Wascheinrichtung, mit der der Formkörper waschbar ist. Hierdurch kann berücksichtigt werden, dass der in Haushalten verwendete Formkörper beziehungsweise Blähsack oder die Bügelpuppe in regelmäßigen Abständen gewaschen wird. Auch dieses Waschen greift üblicherweise das Textilgewebe an und verkürzt in einem gewissen Maß die Lebensdauer des Formkörpers.

Ferner kann eine Bestrahlungseinrichtung vorgesehen sein, mit der der Formkörper mit elektromagnetischer Strahlung bestrahlt wird. Hierdurch kann berücksichtigt werden, dass die Bügelmaschine beispielsweise einem gewissen Maß an UV-Strahlung ausgesetzt ist. In gleicher Weise könnte hierdurch beispielsweise auftretende Infrarotstrahlung oder sichtbares Licht, sofern es das Textilmaterial des Formkörpers wesentliche beeinflusst, berücksichtigt werden.

Des Weiteren kann eine Sprüheinrichtung vorgesehen sein, mit der der Formkörper mit Flüssigkeit besprühbar ist. Dies hat den Zweck, übliche Einsprühvorgänge mit Wasser oder beliebigen anderen Substanzen nachzustellen. Auch dies trägt maßgeblich zur Materialermüdung bei.

Der Formkörper, der ― wie bereits erwähnt ― die Form eines Textilsacks oder einer Textilpuppe annehmen kann, kann auch aus mehreren Materialien unterschiedlicher Luftdurchlässigkeit hergestellt sein. Dies ist beispielsweise notwendig, um zum Bügeln von Knopfleisten von Hemden einen erhöhten Luftdurchsatz gegenüber beispielsweise der Rückenfläche eines Hemds zu gewährleisten, so dass ein gleichmäßiger Bügel- beziehungsweise Trocknungsvorgang erzielt werden kann. Daher müssen die Mess- und Auswerteeinrichtungen entsprechend ausgelegt sein, um die verschiedenen Materialien hinsichtlich ihrer Abnutzung bzw. ihrer veränderten Luftdurchlässigkeit zu bewerten.

Zweckmäßigerweise verfügt die Lebensdauerprüfvorrichtung über ein Steuergerät, mit dem einzelne Komponenten der Vorrichtung zyklisch ansteuerbar sind. Damit können typische Zyklen wie Einsprühen und Bügeln des Bügelguts mehrfach wiederholt automatisch simuliert werden.

Die Vorrichtung zur Bestimmung der Lebensdauer der Bügelvorrichtung weist ferner vorzugsweise mehrere Formkörper auf, deren Lebensdauer parallel bestimmt wird. Damit kann ohne verhältnismäßig großen Mehraufwand eine gewisse statistische Sicherheit über die zu ermittelnden Lebensdauern erhalten werden.

Die vorliegende Erfindung wird nun anhand der beigefügten Zeichnungen näher erläutert, in denen zeigen:
- Figur 1: ein Foto einer Lebensdauerbestimmungsvorrichtung, in der mehrere Säckchen aufgeblasen sind;
- Figur 2: ein Foto der Lebensdauerbestimmungsvorrichtung, in der die Säckchen entleert sind;
- Figur 3: ein Foto der Lebensdauerbestimmungsvorrichtung beim Besprühen; und
- Figur 4: ein Zeitablaufdiagramm, das mehrere Simulationszyklen zur Bestimmung der Lebensdauer der Bügelvorrichtung zeigt.

Die nachfolgenden Ausführungsbeispiele stellen bevorzugte Ausführungsformen der vorliegenden Erfindung dar.

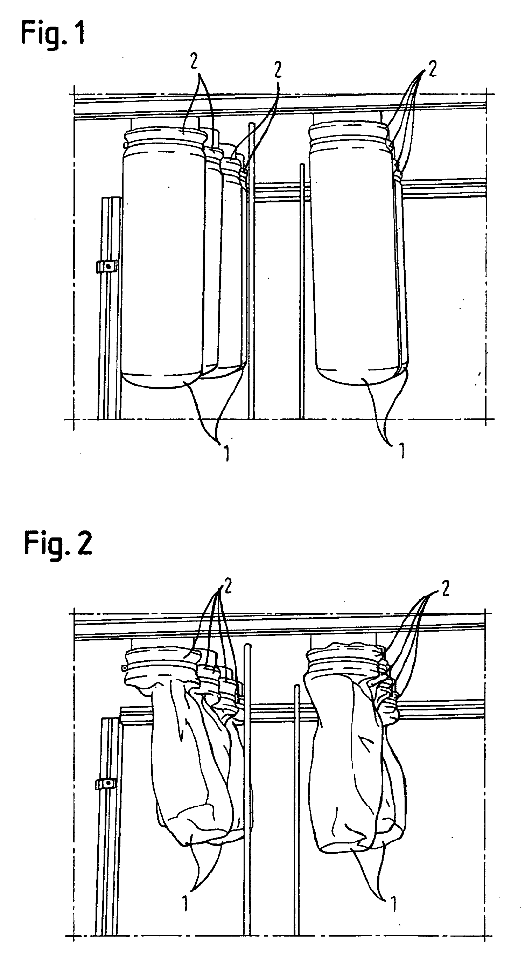

Das Foto in Figur 1 zeigt einen Prüfstand, der aus acht Behandlungsplätzen besteht, wobei die Behandlungsproben, nämlich die Säckchen beziehungsweise Formkörper 1 an geeigneten Stutzen 2 einer Befüllungsvorrichtung hängen beziehungsweise befestigt sind. Bei den Behandlungsproben handelt es sich um zylindrische Säckchen, die aus einem oder mehreren zu prüfenden Materialien zusammengesetzt sind.

Die Säckchen 1 werden zyklisch mit warmer beziehungsweise klimatisierter Luft aufgeblasen und geleert, wie dies in Figur 2 dargestellt ist.

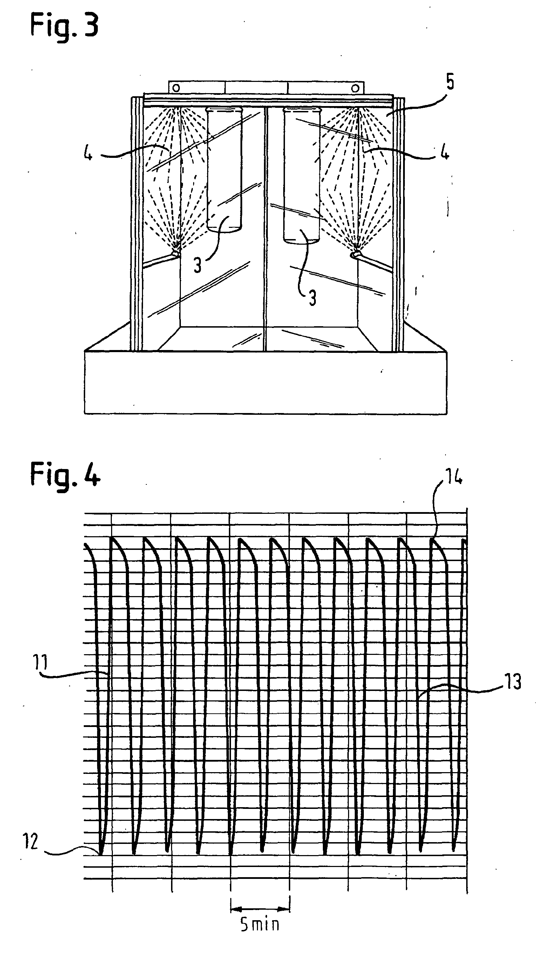

Um die Säckchen wird gemäß Figur 3 ein Schlauch 3 aus Bügelgutmaterial gespannt, um beim Aufblasen die durch das Bügelgut verursachte Reibung zu simulieren, die sich durch die Relativbewegung zwischen dem feststehenden Bügelgut und dem sich befüllenden und entleerenden Säckchen ergibt. Das Bügelgut wird während des Entleerens mit Wasser 4 besprüht. Die Behandlungsproben befinden sich hierfür in einer abgeschlossenen Kammer 5.

In Figur 4 ist die Temperaturkurve während der Behandlungszyklen dargestellt. Die erste in Figur 4 dargestellte Phase 11 entspricht einer Abkühlphase. In ihr wird die Luft aus dem Blähsack beziehungsweise der Bügelpuppe herausgesogen und gleichzeitig erfolgt ein Besprühen mit Wasser und gegebenenfalls entsprechenden Zusatzstoffen. Diese Phase dauert etwa 30 Sekunden. Das Ende der Abkühlphase ist in Figur 4 mit 12 bezeichnet. An diesem Punkt erreicht der Blähsack, der auch als Bügelgutschlauch bezeichnet werden kann, eine Temperatur von ca. 30° C.

Es schließt sich eine Aufblasphase 13 an, in der der Blähsack mit warmer Luft aufgeblasen wird. Der aufgeblasene Zustand bleibt für etwa 120 Sekunden erhalten. Das Ende der Aufblasphase ist in Figur 4 mit dem Bezugszeichen 14 gekennzeichnet. An dieser Stelle erreicht der Blähsack eine Temperatur von ca. 100° C.

Wie ebenfalls der Figur 4 zu entnehmen ist, dauert ein gesamter Zyklus mit Abkühlphase, Aufblasphase und Verweilen im aufgeblasenen Zustand ca. 3 Minuten.

Je nach der zu vermutenden Belastung, der der textile Formkörper beziehungsweise die Bügelpuppe ausgesetzt sein wird, sind noch weitere Belastungen und Simulationen vorzusehen. Da die Bügelpuppen in regelmäßigen Abständen gewaschen werden, muss dies auch bei der Simulation der Lebensdauer erfolgen. Daher wird nach einer vorgegebenen Anzahl an Aufblas- und Entleerungsphasen ein Waschzyklus durchgeführt. Mehrere dieser Waschzyklen führen zu einem nicht unwesentlichen Alterungsprozess der Textilfasern der Bügelpuppe beziehungsweise des Blähsacks.

Darüber hinaus werden die Blähsäcke während der Lebensdauerprüfung auch mit UV-Licht bestrahlt, um die realen Strahlungsbedingungen, in denen die Bügelpuppen üblicherweise betrieben werden, zu simulieren.

Die reale Umgebungsluft für Bügelpuppen im Haushalt ist auch mit gewissen Schadgasen, wie Stickoxiden und schwefelhaltige Verbindungen, belastet. Daher kann die Prüfung in einer Atmosphäre mit einer entsprechenden Schadgaskonzentration erfolgen. Dies kann während des zyklischen Befüllens und Entleerens oder auch vor oder nach dem zyklischen Befüllen und Entleeren in einer separaten Kammer ohne zyklisches Befüllen und Entleeren erfolgen. Darüber hinaus können noch weitere Belastungen, die zur Alterung der textilen Bügelpuppen führen, während der Prüfung vorgenommen werden.

Vor, nach und zwischen den Behandlungen erfolgen diverse Textilprüfungen, um die Wirkung auf das Material zu beobachten. Hier sind zu nennen: Luftdurchlässigkeit, Höchstzugkraft, Höchstzugkraftdehnung und alle weiteren Merkmale, die für die Tauglichkeit des textilen Bauteils wichtig sind.

Nachfolgend wird der Ablauf und Prüfplan einer konkreten Simulation näher dargestellt, wie er im Labor bereits durchgeführt wurde. Dabei richtet sich der Prüfplan nach der Art der vorgesehenen Belastung des Geräts und der angestrebten Lebensdauer. Bei dem hier vorgestellten Prüfplan gemäß der folgenden Tabelle wurde davon ausgegangen, dass der Blähsack im Jahr ca. 1000-mal benutzt und zwei- bis dreimal gewaschen wird.

Die Belastungssimulation erfolgt in Blöcken von je 1/3 der Lebensdauer. Der letzte Block wurde dreifach unterteilt, um am Ende der Lebensdauer eine genauere Bestimmung vornehmen zu können.

**Tabelle:**

| Prüfplan über eine Simulation von 10 Jahren. | | | |
|---|---|---|---|
| Zustand/Behandlung | Zyklen Differenz zum Vorzustand | Gesamtzyklen kumuliert | Prüfungen |
| Original | | | LD (Luftdurchlässigkeit) |
| 1 x Waschen | | | LD, KD (Kraft/Dehnung, Reißfestigkeit) |
| 1/3 der Gesamt - Kälte/Wärme ― Behandlung/Schadgas KWB | 1/3 | 1/3 | LD bei Bedarf |
| 3200 x Bügeln | 3200 | 3200 | LD bei Bedarf |
| 7 x Waschen | 7 | 8 | LD, KD, KF (Knickfestigkeit) |
| 1/3 der Gesamt-KWB | 1/3 | 2/3 | LD bei Bedarf |
| 3200 x Bügeln | 3200 | 6400 | LD bei Bedarf |
| 8 x Waschen | 8 | 16 | LD, KD, KF |
| 1/3 der Gesamt-KWB | 1/3 | 3/3 | LD bei Bedarf |
| 800 x Bügeln | 800 | 7200 | LD |
| 2 x Waschen | 2 | 18 | LD |
| 800 x Bügeln | 800 | 8000 | LD |
| 2 x Waschen | 2 | 20 | LD |
| 800 x Bügeln | 800 | 8800 | LD |
| 2 x Waschen | 2 | 22 | LD |
| 800 x Bügeln | 800 | 9600 | LD |
| 2 x Waschen | 2 | 24 | LD, KD, KF |

Gemäß der Tabelle beginnt die Lebensdauerprüfung durch eine Luftdurchlässigkeitsprüfung (LD) des Originalblähsacks. Anschließend erfolgt ein erstes Vorwaschen um gegebenenfalls Appreturen des Herstellers aus dem Textilgewebe zu entfernen. Anschließend erfolgt wieder eine LD-Prüfung und eine Kraft/Dehnungs-Messung (KD), bei der die Reißfestigkeit vor den weiteren Belastungen im Originalzustand geprüft wird.

Als erste Belastung erfolgt eine Kälte/Wärme-Behandlung (KWB) mit zusätzlicher Schadgasbelastung. Diese KWB-Belastung entspricht etwa 1/3 der realen Belastung während einer angestrebten Lebensdauer. Nach dieser KWB-Belastung erfolgt bei Bedarf eine LD-Prüfung.

Im Anschluss daran erfolgt die eigentliche Bügelbelastung des ersten Blocks. Hierbei werden 3200 Bügelzyklen am Stück durchgeführt, wobei jeder dieser Zyklen dem im Zusammenhang mit Figur 4 beschriebenen Zyklus entspricht. Nach Abschluss der Bügelvorgänge folgt je nach Bedarf eine LD-Prüfung.

Zum Abschluss des ersten Blocks erfolgen die typischerweise durchgeführten Waschvorgänge. Nachdem ein Waschvorgang in dem ersten Block bereits durchgeführt worden ist, erfolgen noch sieben weitere Waschdurchgänge. Im Anschluss daran erfolgt eine LD-, KD- und eine Knickfestigkeitsprüfung (KF). Damit ist der erste Block abgeschlossen.

Im zweiten Block erfolgen wie im ersten Block in einem ersten Belastungsschritt 1/3 der Gesamt-KWB, in einem zweiten Schritt wiederum 3200 Bügelvorgänge und in einem dritten Schritt acht Waschvorgänge.

Der dritte und letzte Block beginnt im ersten Schritt erneut mit 1/3 der Gesamt-KWB. Die anschließenden Bügel- und Waschzyklen sind jedoch in vier Einzelblöcke mit Bügel- und Waschphasen unterteilt. Nach jeder Phase erfolgt eine LD-Prüfung, damit die Lebensdauer nun genauer bestimmt werden kann. Am Ende erfolgt zusätzlich eine KD- und KF-Prüfung.

## Patentansprüche

1. Vorrichtung zur Bestimmung der Lebensdauer eines befüllbaren, textilen Formkörpers (1) für eine Bügelvorrichtung,
**gekennzeichnet durch**
eine Befüllungseinrichtung, mit der der befüllbarene Formkörper (1) mit einem Medium, insbesondere Luft, wiederholt befüllbar und leerbar ist.

2. Vorrichtung nach Anspruch 1, die eine Messeinrichtung zum Messen mindestens einer Materialeigenschaft eines Bügelguts aufweist und/oder eine Messeinrichtung zum Messen von Prozessbedingungen, wie Temperatur, Druck oder Feuchtigkeit des Mediums aufweist.

3. Vorrichtung nach Anspruch 2, wobei die Materialeigenschaft die Luftdurchlässigkeit, die Höchstzugkraft oder die Höchstzugkraftdehnung ist.

4. Vorrichtung nach Anspruch 2 oder 3, die eine Auswerteeinrichtung aufweist, mit der aus der gemessenen, mindestens einen Materialeigenschaft die Lebensdauer des Formkörpers bestimmbar ist.

5. Vorrichtung nach einem der Ansprüche 1 bis 4, die eine Befülleinrichtung aufweist, mit der die einzufüllende Luft klimatisierbar ist.

6. Vorrichtung nach Anspruch 5, wobei die Luft mit Gasen, insbesondere Schadgasen oder Dämpfen versetzbar ist.

7. Vorrichtung nach einem der Ansprüche 1 bis 6, wobei auf dem Formkörper (1) ein Bügelgut (3) aufgebracht ist, so dass durch das Befüllen und Entleeren des Formkörpers eine Relativbewegung gegenüber dem Formkörper erzeugbar ist.

8. Vorrichtung nach einem der Ansprüche 1 bis 7, die eine Wascheinrichtung aufweist, mit der der Formkörper (1) waschbar ist.

9. Vorrichtung nach einem der Ansprüche 1 bis 8, die eine Bestrahlungseinrichtung aufweist, mit der der Formkörper (1) mit elektromagnetischer Strahlung bestrahlbar ist.

10. Vorrichtung nach einem der Ansprüche 1 bis 9, die eine Sprüheinrichtung aufweist, mit der der Formkörper (1) mit Flüssigkeit (4) besprühbar ist.

11. Vorrichtung nach einem der Ansprüche 1 bis 10, wobei der Formkörper (1) ein Textilsack oder eine Textilpuppe ist.

12. Vorrichtung nach einem der Ansprüche 1 bis 11, wobei der Formkörper (1) aus einem einheitlichen oder aus mehreren unterschiedlichen Materialien besteht.

13. Vorrichtung nach einem der Ansprüche 1 bis 12, die ein Steuergerät aufweist, mit dem einzelne Komponenten der Vorrichtung zyklisch ansteuerbar sind.

14. Vorrichtung nach einem der Ansprüche 1 bis 13, die mehrere Formkörper (1) aufweist, deren Lebensdauern parallel bestimmbar sind.

15. Verfahren zur Bestimmung der Lebensdauer eines befüllbaren, textilen Formkörpers für eine Bügelvorrichtung,
**gekennzeichnet durch**
wiederholtes Befüllen und Entleeren des befüllbaren Formkörpers (1) mit einem Medium, insbesondere Luft.

16. Verfahren nach Anspruch 15, wobei mindestens eine Materialeigenschaft des Formkörpers (1) gemessen wird und/oder eine Prozessbedingungen, wie Temperatur, Druck oder Feuchtigkeit des Mediums gemessen wird.

17. Verfahren nach Anspruch 16, wobei die mindestens eine Materialeigenschaft die Luftdurchlässigkeit, die Höchstzugkraft oder die Höchstzugkraftdehnung ist.

18. Verfahren nach einem der Ansprüche 15 bis 17, wobei die in den Formkörper (1) gefüllte Luft klimatisiert wird.

19. Verfahren nach einem der Ansprüche 15 bis 18, wobei die in den Formkörper (1) gefüllte Luft mit Gasen, insbesondere Schadgasen, oder Dämpfen versetzt wird.

20. Verfahren nach einem der Ansprüche 15 bis 19, wobei auf den Formkörper (1) ein Bügelgut (3) aufgebracht wird, so dass durch das Befüllen und Entleeren des Formkörpers eine Relativbewegung gegenüber dem Formkörper erzeugt wird.

21. Verfahren nach einem der Ansprüche 15 bis 20, wobei der Formkörper (1) gewaschen wird.

22. Verfahren nach einem der Ansprüche 15 bis 21, wobei der Formkörper (1) mit elektromagnetischer Strahlung bestrahlt wird.

23. Verfahren nach einem der Ansprüche 15 bis 22, wobei der Formkörper (1) mit Flüssigkeit (4) besprüht wird.

24. Verfahren nach einem der Ansprüche 15 bis 23, wobei der Formkörper (1) ein Textilsack oder eine Textilpuppe ist.

25. Verfahren nach einem der Ansprüche 15 bis 24, wobei der Formkörper (1) aus einem einheitlichen oder aus mehreren unterschiedlichen Materialien gefertigt ist.

26. Verfahren nach einem der Ansprüche 15 bis 25, wobei die Lebensdauer mehrerer Formkörper (1) parallel bestimmt wird.
